# EUROPEAN PATENT APPLICATION

(11) **EP 2 345 725 A1**
(43) Date of publication of application: **20.07.2011**
(21) Application number: 09761236.0
(22) Date of filing: 02.06.2009
(51) Int. Cl.: C12N 15/53, C12N 9/02, C12Q 1/68, C12P 23/00

(54) **USE OF GENE IN THE SYNTHESIS OF ASTAXANTHIN**

(30) Priority: 09.06.2008 CL 16992008
(71) Applicant: Cifuentes Guzman, Victor, 8330015 Santiago (CL)
(72) Inventor: ALCAINO GORMAN, Jennifer, 8330015 Santiago (CL)
(74) Representative: Carpintero Lopez, Francisco
(86) International application number: PCT/CL2009/000001
(87) International publication number: WO 2009/149578

(57) **Abstract**

A DNA sequence which encodes for an enzyme having cytochrome P450 reductase activity which acts as donor of electrons for the reaction catalysed by the enzyme astaxanthin synthase and which converts beta-carotene into astaxanthin, preferably in *X. dendrorhous.*

## Description

### FIELD OF THE INVENTION

Dendrorhous Xanthophyllomyces basidiomycete yeast synthesizes carotenoids, mainly xanthophylls astaxanthin, of great commercial interest due to its use as a food supplement in the aquaculture industry.

The present invention describes the genetic material that encodes the polypeptide, which has cytochrome P450 reductase activity and participates in the synthesis of astaxanthin from beta-carotene, acting as electron donor in the reaction catalyzed by astaxanthin synthase.

### BACKGROUND OF THE INVENTION

Carotenoids are a group of natural pigments produced principally by plants, yeast and microalgae. Particularly the xanthophyll astaxanthin is a compound widely found in nature, this has great commercial importance because it is used as a food additive to pigment the trout and salmon flesh in aquaculture (Johnson et al. 1977). On the other hand and due to its high antioxidant activity, there have been benefits to human health, for example, in preventing cardiovascular disease, supports the immune system, bioactivity against *Helicobacter pylori* and prevention of ocular cataracts (Higuera-Ciapara and et al. 2006).

Originally, the preparation of this compound was made directly from crustaceans (bodies rich in this pigment), at present production is mainly by chemical synthesis, some examples are described in US Patents US 4.245.109, US 5.210.314, US 5.625.099 and US5.654.488 patents.

Another way of obtaining astaxanthin is the biosynthesis and extraction from microorganisms, in this sense the most important is the use of microalgae (*Haematococcus* pluvial) and yeast *(Xanthophyllomyces dendrorhous*). Growth, production and extraction of astaxanthin in microalgae has been described in several patents, including Canadian patent CN 1.966.660, and the Japanese patent JP 2007/222168, application KR2005/0005341, and the Mexican application MX/PA05/007801, however, when comparing the astaxanthin produced by *Haematococcus pluvialis* with dendrorhous *Xanthophyllomyces* obtained, there are important differences. In freshwater, unicellular green microalgae *H. pluvialis* has been recognized for many years as an accumulator of the carotenoid astaxanthin, under certain stress conditions such as high light intensity and/or nitrogen limitation, cells of the algae form cysts and accumulate high amounts of astaxanthin (>1% dry weight) (Margalith, 1999), changing its color from green to red.

This microalgae has recently received much attention due to their ability to synthesize and accumulate large quantities of astaxanthin during and after their growth phase (Margalith, 1999). However, the use *H. pluvialis* as a source of astaxanthin was poor due to the production of pigments of lower quality than that required commercially, given the high percentage of sterification of astaxanthin obtained (Sommer et al 1991). In this sense, astaxanthin produced by the yeast *Xanthophyllomyces* dendrorhous, is 100% free (not sterified) and comprises the optical isomer 3R, 3R' and geometric isomer trans, which gives it a comparative advantage on produced by the microalgae (Andrewes and Starr, 1976). Although microalgae are the natural source with the highest astaxanthin content known, the interest for the commercial exploitation of these organisms has not been sufficient due to high rates of contamination with protozoa and less development of culture technologies compared with heterotrophic organisms such as yeast.

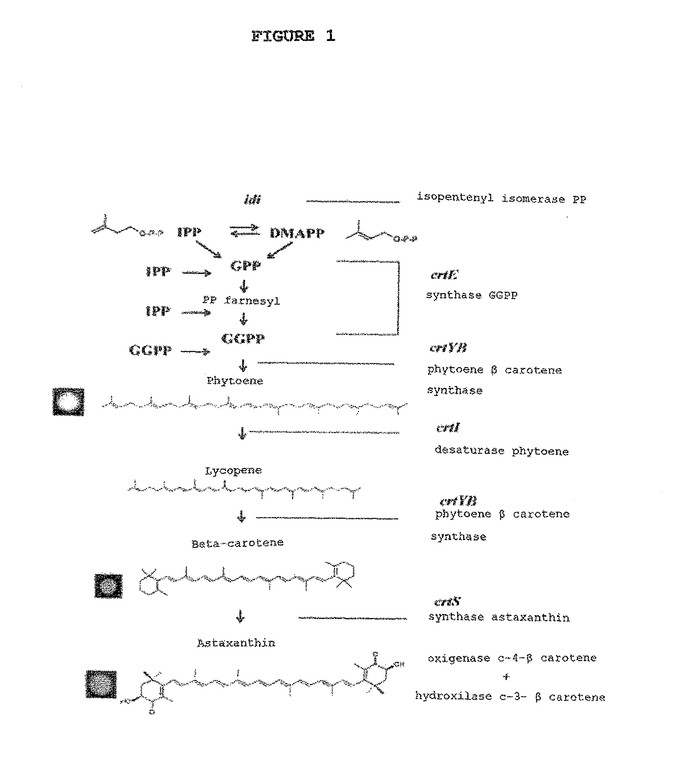

The biosynthetic pathway of carotenoids pigments has been extensively studied in yeast, Figure 1 shows the route identified for X dendrorhous and detail those genes that have been isolated, sequenced, characterized and published (Kajiwara et al., 1997, Niklitschek et al., 2008, Green et al., 1999a; Alcaino, 2002, Green et al., 1999b; León, 2000). In the formation of astaxanthin from beta-carotene requires two enzymatic activities: ketolase incorporating a keto group in positions 4 and 4' of the molecule and beta-carotene hydroxylase which introduces a hydroxyl group at positions 3 and 3 'beta-carotene (Cunningham and Gantt 1998, Linden 1999). Unlike other agencies where two independent genes involved in this step, X. dendrorhous CRTS has isolated a gene that codes for an enzyme astaxanthin synthase which has both activities (EP 1,035,206; Ojima et al., 2006, Alvarez et al., 2006). From the deduced amino acid sequence of the gene by CRTs and bioinformatic analysis, we determined that astaxanthin synthase enzyme X dendrorhous belongs to the family of cytochrome P450 monooxygenases proteins (Ojima et al., 2006).

For the oxygen insertion into the substrate, the enzyme cytochrome P450 monooxygenases type requires an electron donor system in eukaryotes is a protein cytochrome P450 reductase (CPR) (van den Brink et al., 1998). Additionally, Ojima et al (2006) described a job in which *Escherichia coli* was transformed with 3 plasmids compatible with each other, one of the plasmids containing the genes necessary for the synthesis of beta-carotene, another containing the gene X CRTS dendrorhous and the third contained a gene for cytochrome P450 reductase in *Saccharomyces cerevisiae,* this system was only possible to obtain oxygenated derivatives of beta-carotene, but no astaxanthin. From these results we can infer the need for the sequence of cytochrome P450 reductase appropriate to complement the biosynthetic pathway in the system.

In this regard, it has not yet been isolated and sequenced, the gene coding for the cytochrome P450 reductase from X. dendrorhous, nor has demonstrated its involvement in the synthesis of astaxanthin.

This invention describes the crtR gene sequence encoding the polypeptide and which has Cytochrome P450 reductase activity of X. dendrorhous. This invention provides technical tools for transforming microorganisms and is enhancing its natural ability to produce astaxanthin or creating new pathways in other organisms carotenogenic not allow the production of this pigment in a commercially attractive form.

### DESCRIPTION OF THE INVENTION

The present invention relates to genetic material that encodes the enzyme cytochrome P450 reductase (CPR), understanding the genetic material and nucleic acid sequence type genomic DNA, complementary DNA, RNA messenger and the respective allelic variants that encode a polypeptide with CPR activity, useful for the production of astaxanthin by the transformation of host organisms that may or may not be natural producers of this carotenoid.

### BRIEF DESCRIPTION OF THE FIGURES

Figure 1, main steps of the biosynthetic pathway of astaxanthin in X. dendrorhous. It points out the structural genes of the pathway have been cloned and sequenced. It displays the color of the colonies that accumulate intermediates in the carotenoid biosynthetic pathway. The color is an indicator of the type of pigment being obtained at different stages of synthesis.
Figure 2, genomic DNA sequence (SEQ1) CRTR gene X. dendrorhous. The underlined sequence fragments corresponding to exons of the gene. In bold and underlined nucleic acids highlights those sites identified as donors and receivers for joint processes. Indicates the restriction sites for BamHI enzyme.
Figure 3, Comparison of nucleotide sequences of genes for NADPH-cytochrome P450 reductase from different fungi *C_echinulata*: *Cunninghamella echinulata (Zygomycetes)* No access GenBank: AF195660. *S-cerevisiae: Saccharomyces cerevisiae* (Ascomycete) GenBank Access No.: D13788. *R-minuta: Rhodotorula minuta (Basidiomycete)* No access GenBank: AB055119. Gray line shows where some of the primers described in Table 1.
Figure 4, complementary DNA sequence (SEQ2) for the crtR gene coding region and translation into amino acid sequence (SEQ3) with one-letter code for each amino acid. In italics: crtR gene Exon 1. In black: crtR gene Exon 2. Underlined sequence: Exon 3 crtR gene.
Figure 5 Amino acid sequence deduced from the crtR gene coding region. In black are highlighted and underlined conserved domains of the protein, in order are: transmembrane domain, FMN binding domain, FAD binding domain and binding domain of NAD (P) H, identified by BlastP program.
Figure 6 Outline of the process of obtaining construction designed for crtR gene deletion in wild strains CBS6938 UCD67-385 and X dendrorhous.
Figure 7, Schematic of the events of crtR gene mutation by homologous recombination in wild strains UCD67-385 (385) and CBS6938 (CBS) of X. dendrorhous, where A shows the transformation of the strain UCD67-385. and B shows CBS69388 transformation strain.
Figure 8, wild yeast culture (UCD 67-385 and CBS 6938) and transformants (T13, derived from UCD 67-385 and CBSTr: derived from CBS 6938) in a petri dish with YM-agar culture medium.
Figure 9, Pigments present in the wild yeasts (UCD 67-385 and CBS 6938) and transformed (T13 and CBSTr).
   (A) HPLC chromatograms (465 nm). In each peak indicates the corresponding carotenoid, NI: Not Identified.
   (B) Graph showing a comparison of the pigments produced by wild strains and their respective mutant, expressed in ppm (parts per million) of each detected pigment.
Figure 10 gel PCR reaction of wild type and transformants. λ: molecular size standard bacteriophage λ DNA digested with enzyme HindIII (bands of 23.1, 9.4, 6.6, 4.4, 2.3, 2.0 and 0.6 kb)
   **A:** PCR of UCD67-385 and T13 transformer. Path: (1): DNA 385 + gpd rev+SEQ50, (2): DNA T13 + gpd rev+SEQ50, (3) H₂O + gpd rev+SEQ50, (4): DNA 385 + SEQ14+Pef fwd, (5): DNA T13 + SEQ14+Pef fwd, (6): H₂O + SEQ14+Pef fwd, (7): DNA 385 + SEQ14+SEQ50, (8): DNA T13 + SEQ14+SEQ50, (9): H₂O + SEQ14+SEQ50, (10): DNA 385 + SEQ14+SEQ15, (II): DNA T13+ SEQ14+SEQ15 y (12): H₂O+ SEQ14+SEQ15.
   **B:** PCR of CBS6938 and CBSTr transformer. Path: (1): DNA CBS + Pef fwd+SEQ35, (2): DNA CBSTr + Pef fwd+SEQ35, (3): H₂O + Pef fwd+SEQ35, (4): DNA CBS + gpd rev+SEQ30, (5): DNA CBSTr + gpd rev+SEQ30 y (6): H₂O + gpd rev+SEQ30.
   **C:** PCR of CBSTr transformer. Path: (1): DNA CBSTr + SEQ75+hygSecR, (2): DNA CBSTr + HF+SEQ35, (3): DNA CBSTr + HygSecF+M13F, (4): DNA CBSTr + M13R+SEQ15, (5): DNA CBSTr + SEQ20+SEQ50 y (6): DNA CBSTr + SEQ30+SEQ50.

### DETAILED DESCRIPTION OF THE INVENTION

The present invention relates to a gene and an enzyme that is involved in the final stage of the synthesis of astaxanthin from beta-carotene.

The present invention provides a genomic DNA sequence (gene crtR) and complementary DNA encoding an enzyme with cytochrome P450 reductase (CPR) is responsible for delivering the electrons needed for the incorporation of keto and hydroxyl groups on the molecule of beta-carotene astaxanthin for this reaction is catalyzed by the enzyme astaxanthin synthase.

The present invention relates to DNA sequences of gene X ACWP dendrorhous, in its preferred embodiment sequences of genomic and complementary DNA, as well as fragments of the gene, and these semi-degenerate or not.

The present invention relates to a genomic DNA sequence of the gene crtR, its complementary strand, their allelic variants and sequences represented with addition, insertion, deletion and/or replacement of one or more nucleotides that encode a polypeptide having enzymatic activity CPR.

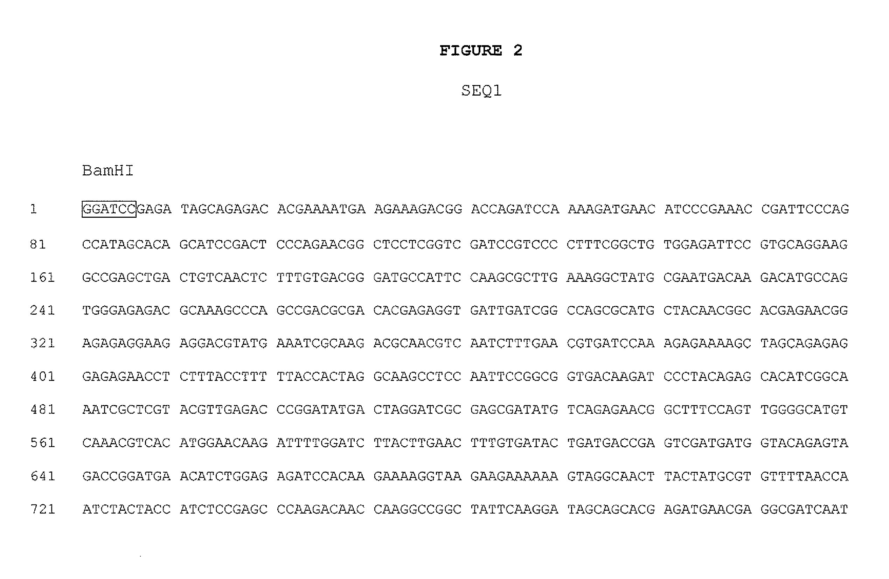

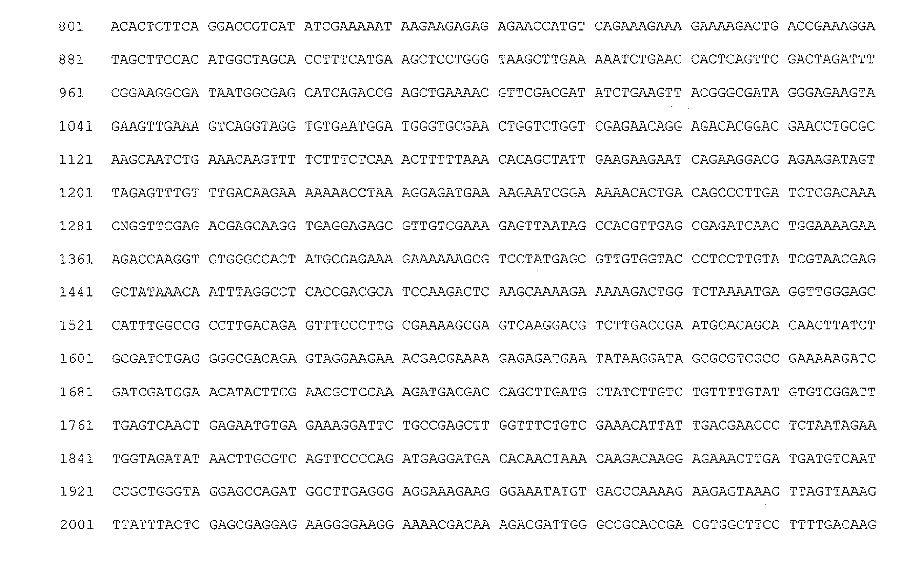

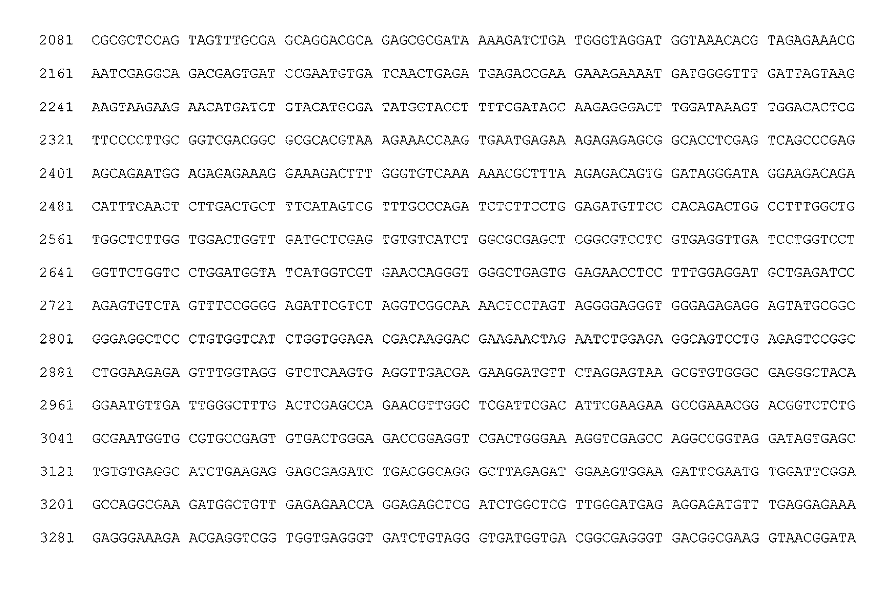

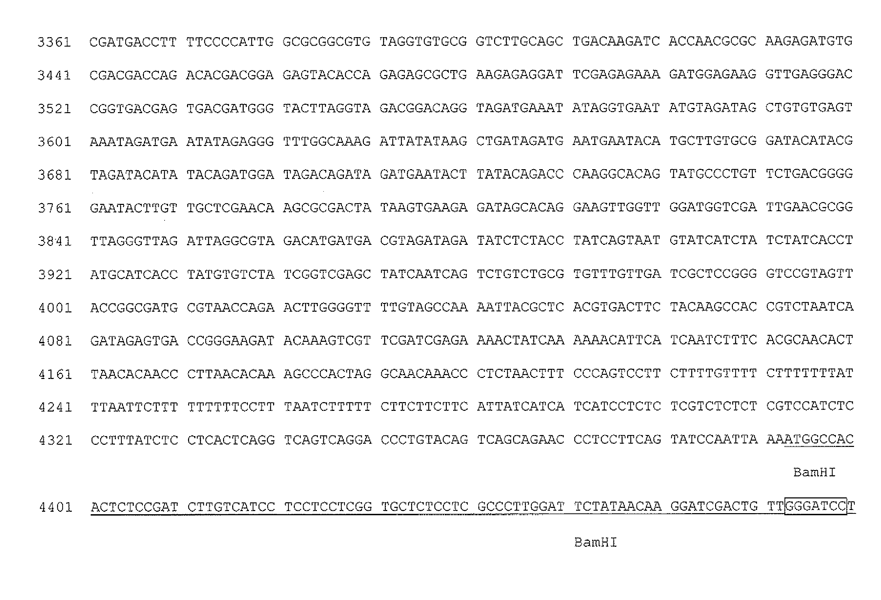

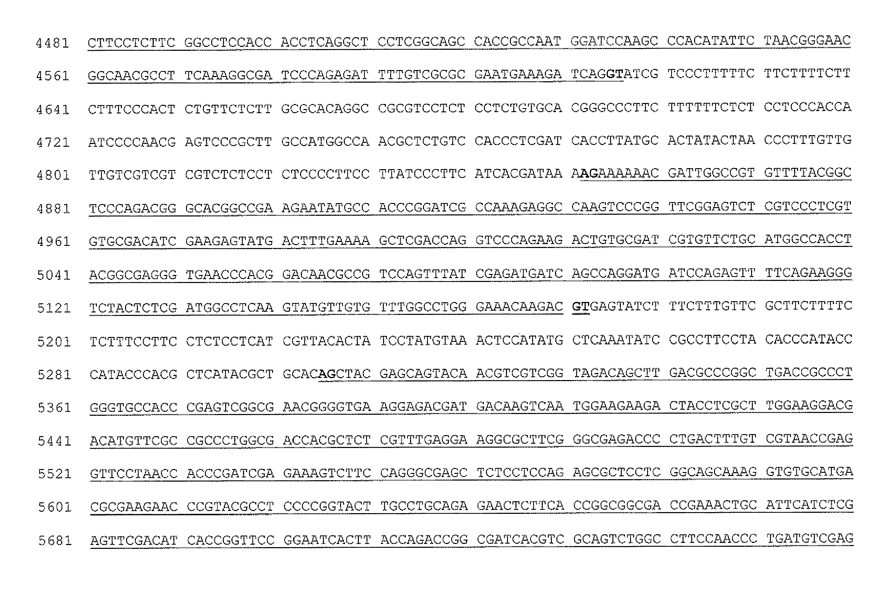

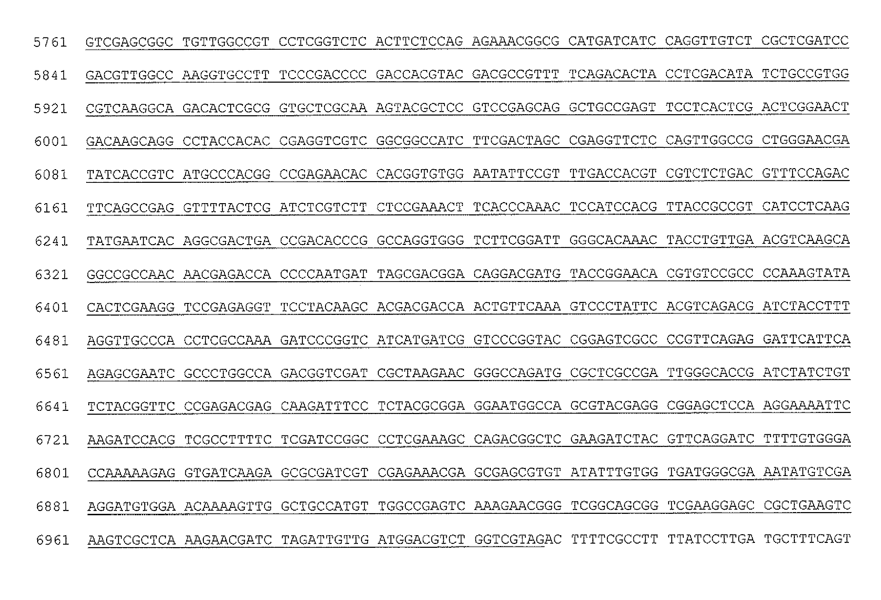

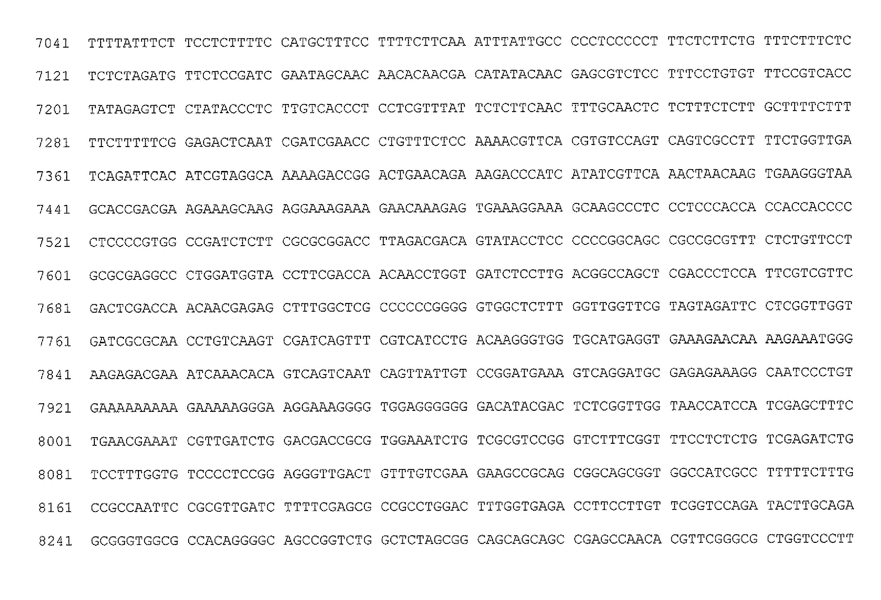

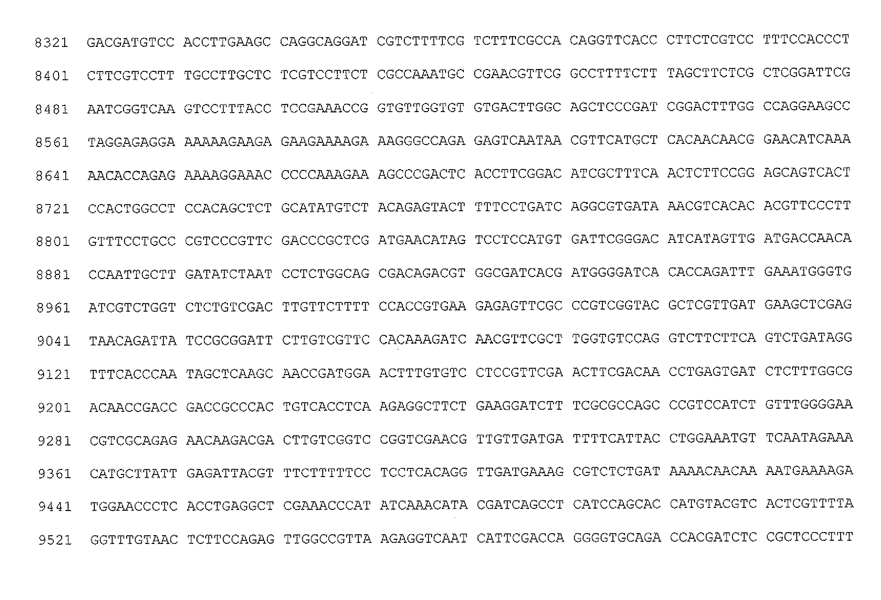

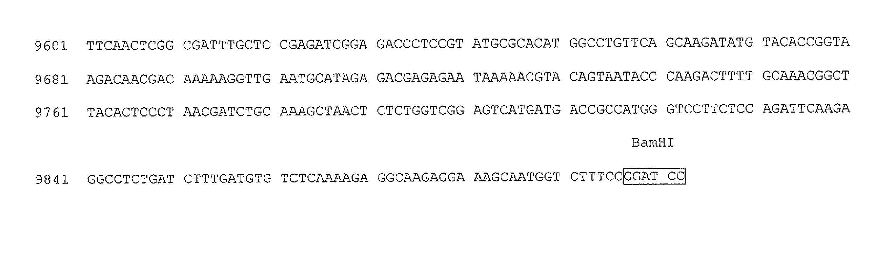

Because the genetic code is degenerate, this invention also provides nucleic acid sequences that hybridize with the crtR gene under standard stringency conditions, such as described by Sambrook et al 2001. In its preferred embodiment, the DNA sequence corresponding to the SEQ1 and features come from X. dendrorhous and have 9.903 base pairs, within it are identified nucleic acids described as donor and recipient sites for splicing (splicing), said nucleic acids are marked in black and outlined in the SEQ1 (Figure 2). The genomic DNA sequence of this gene is characterized by 3 segments of the sequence corresponding to exons highlighted in the SEQ1.

This invention is also considered a complementary DNA sequence, its complementary strand and messenger RNA sequence that generates it, their allelic variants and sequences represented with addition, insertion, deletion and /or replacement of one or more nucleotides that encodes a polypeptide CPR has enzymatic activity.

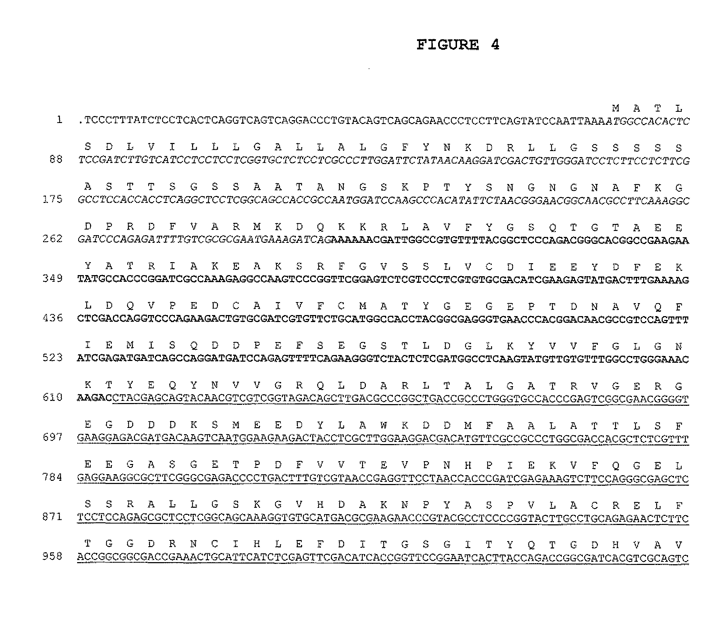

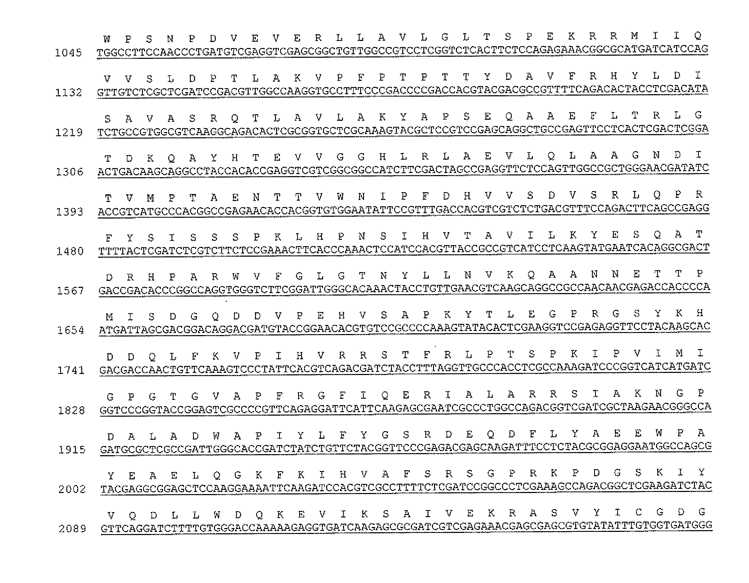

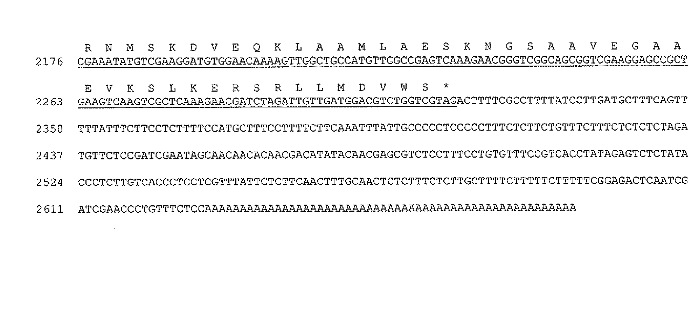

Because the genetic code is degenerate, this invention also provides nucleic acid sequences that hybridize to the complement of the CRTR gene under standard stringency conditions, such as described by Sambrook et al. In its preferred embodiment the cDNA is synthesized from the mRNAs generated from the CRTR gene and refers to the SEQ2 (Figure 4), this cDNA represents the CRTR gene encoding segment and has a length of 2.241 base pairs.

The invention also relates to methods for obtaining DNA sequences of the crtR gene X. dendrorhous. In a first mode or stage this method corresponds to the identification of genomic DNA of the gene of interest, or part thereof, using semi-degenerate fragments. Additionally, in another form or consecutive stage, you identify the gene of interest sequence fragments isolated from the previous stage. Preferably in the first stage are used consensus sequences of conserved segments between genes equivalent to interest from other species.

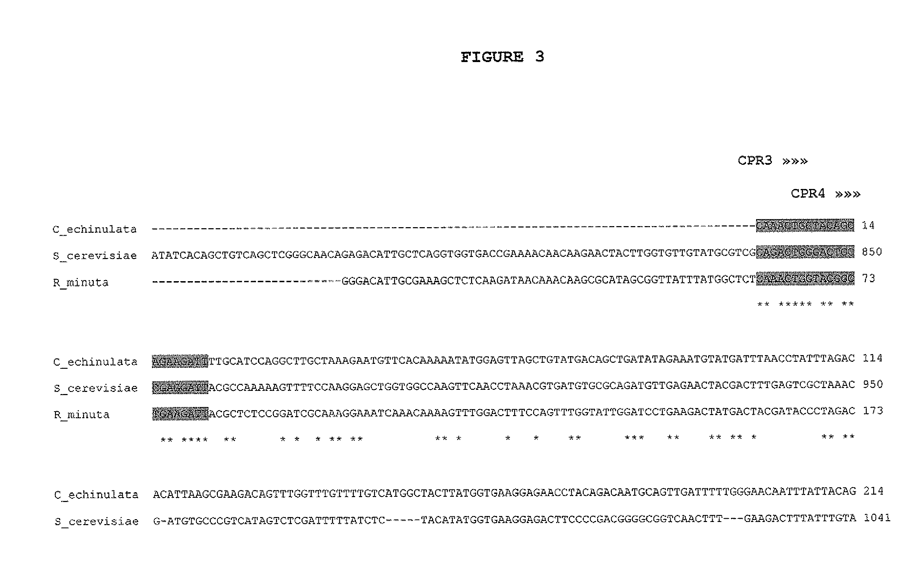

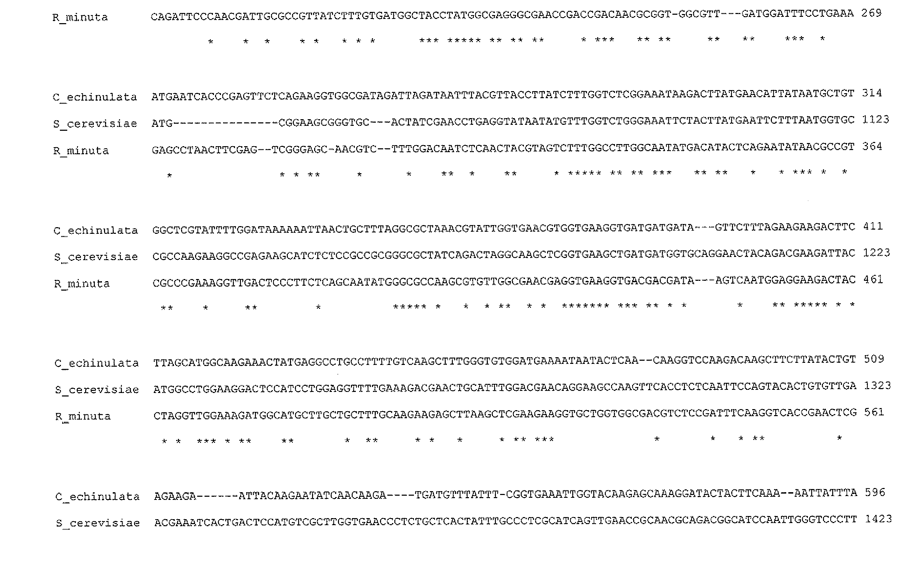

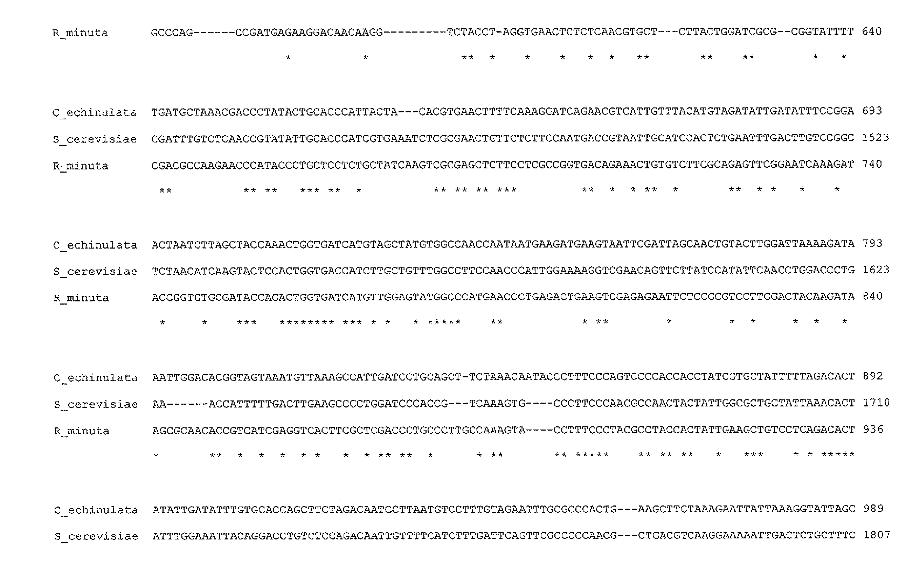

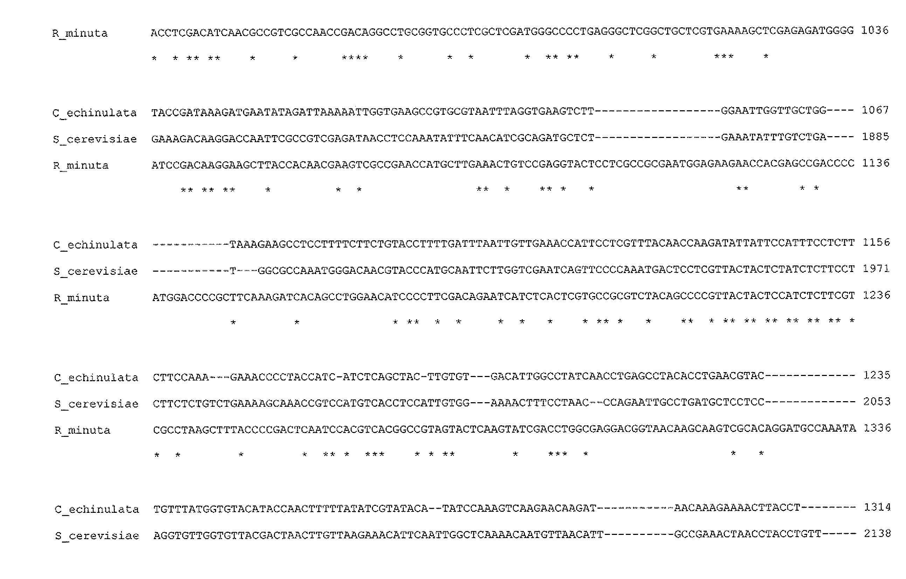

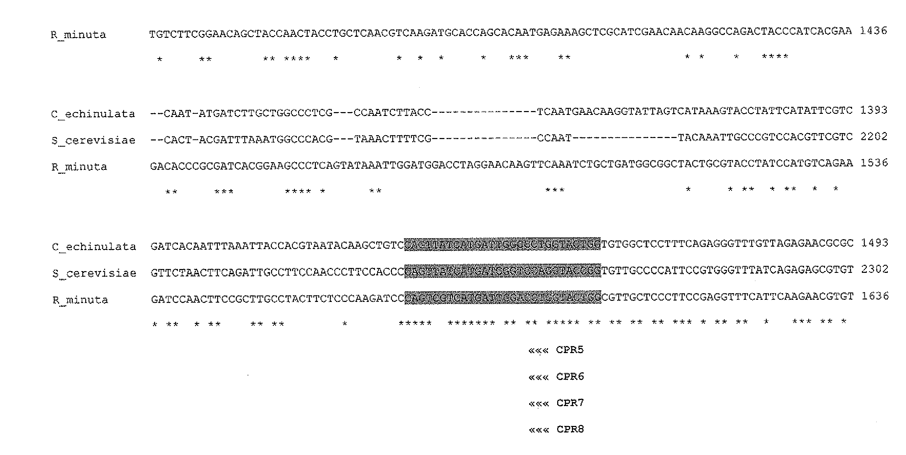

In a preferred embodiment, the invention comprises the comparison using bioinformatic techniques known sequences of cytochrome P450 reductase genes of other yeasts, the identification of conserved segments and design, according to them, short fragments of nucleic acids (15 and 30 bp), useful for the identification and amplification of DNA sequences. In its preferred embodiment, is designed DNA fragments described in Table 1 (SEQ4 to SEQ9). These are semi-degenerate primers and consider the characters R, K, D, H, W, and as representations of the following groups of nucleic acids: R = G or A, K = G or T, D = G or A or T , H = A or T or C, W = A or T, Y = T or C, the location of these primers within the crtR gene genomic sequence of the species considered for this design is described in Figure 3. These fragments were used to amplify part of the CRTR gene from genomic DNA of X. dendrorhous by PCR (Polimerase Chain Reaction). In the second stage are designed degenerate DNA fragments of Table 1 (SEQ10 to SEQ106).

In particular the primers from SEQ10 to SEQ13 were used to identify DNA clones from genomic and complementary DNA of X. dendrorhous carriers CRTR gene fragments by PCR technique.

The rest of the primers above were used to full gene sequencing crtR.

**Table 1: DNA fragments useful for the identification and crtR gene amplification.**

| **Sequence N°** | **Name** | **Sequence 5' - 3'** | **orientation** | **Position related SEQ1 (pb)** |
|---|---|---|---|---|
| SEQ4 | CPR 3 | CARACTGGKACDGCHGARGATT | Direct | 4884- 4905 |
| SEQ5 | CPR 4 | CAAACTGGTACGGCTGAAGATT | Direct | 4884- 4905 |
| SEQ6 | CPR 5 | WGGDCCRATCATGAYRACTGG | Reverse | 6505- 6525 |
| SEQ7 | CPR 6 | AGGTCCAATCATGACGACTGG | Reverse | 6505- 6525 |
| SEQ8 | CPR 7 | CRGTACCWGGDCCRATCATGA | Reverse | 6512- 6532 |
| SEQ9 | CPR 8 | CCAGTACCAGGTCCAATCATGA | Reverse | 6512- 6533 |
| SEQ10 | CPR 9 | GGATCCGCGACATCGAAGAGTATG AC | Direct | 4963- 4982 |
| SEQ11 | CPR10 | GGATCCAGACTGTGCGATCGTGTT CT | Direct | 5009- 5028 |
| SEQ12 | CPR11 | GGATCCAAGCTGTCTACCGACGAC G | Reverse | 5322- 5340 |
| SEQ13 | CPR12 | GGATCCCTTCCAAGCGAGGTAGTC TT | Reverse | 5417- 5436 |
| SEQ14 | CPR 3A | TACAACGTCGTCGGTAGACA | Direct | 5317- 5336 |
| SEQ15 | CPR 7A | TATTCCACACCGTGGTGTTC | Reverse | 6105- 6124 |
| SEQ16 | CPR3B | TACGAGCAGTACAACGTCGT | Direct | 5308- 5327 |
| SEQ17 | CPR7B | TATTCCACACCGTGGTGTTC | Reverse | 6105- 6124 |
| SEQ18 | CPR3C | GTATGTTGTGTTTGGCCTGG | Direct | 5141- 5160 |
| SEQ19 | CPR7C | TCAACAGGTAGTTTGTGCCC | Reverse | 6291- 6310 |
| SEQ20 | CPR3D | GGTCTCACTTCTCCAGAGAA | Direct | 5785- 5804 |
| SEQ21 | CPR7D | ATGAATGCAGTTTCGGTCGC | Reverse | 5657- 5676 |
| SEQ22 | CPR3E | GCTCAAATATCCGCCTTCCT | Direct | 5250- 5269 |
| SEQ23 | CPR7E | TCGCCTGTGATTCATACTTGA | Reverse | 6236- 6256 |
| SEQ24 | CPR1.3A | GAAGAATATGCCACCCGGAT | Direct | 4899- 4918 |
| SEQ25 | CPR1.3B | ACCTAAAACGAGTGACGTAC | Reverse | 9504- 9523 |
| SEQ26 | CPR1.3C | GGCCTCTGATCTTTGATGTG | Reverse | 9841- 9860 |
| SEQ27 | CPR385A | GAATATGTGGGCTTGGATCC | Reverse | 4531- 4550 |
| SEQ28 | CPR385B | CCTTTGAAGGCGTTGCCGTT | Reverse | 4558- 4577 |
| SEQ29 | CPR 12A | TACAACGTCGTCGGTAGACA | Direct | 5317- 5336 |
| SEQ30 | CPR 12B | TACTCTTCGATGTCGCACAC | Reverse | 4959- 4978 |
| SEQ31 | CPR 13 | AGAAGACTGTGCGATCGTGT | Direct | 5006- 5025 |
| SEQ32 | CPR16 | GCCGTTTTCAGACACTACCT | Direct | 5884- 5903 |
| SEQ33 | CPR20 | TCTCGTCTCTATGCATTCAAC | Reverse | 9697- 9717 |
| SEQ34 | CPR27 | TGGGCACAAACTACCTGTTG | Direct | 6290- 6309 |
| SEQ35 | CPR28 | GTCGCCAAAGAGATCACTCA | Reverse | 9183- 9202 |
| SEQ36 | CPR31 | TCAAGCAATTGGTGTTGGTC | Reverse | 8873- 8892 |
| SEQ37 | CPR32 | GGTACGCTCGTTGATGAAGC | Direct | 9016- 9035 |
| SEQ38 | CPR34 | CCCGTTCAGAGGATTCATTC | Direct | 6540- 6559 |
| SEQ39 | CPR35 | TAGCGATCGACCGTCTGG | Reverse | 6578- 6595 |
| SEQ40 | CPR36 | AGGGTGGACAGAGCGTTG | Reverse | 4749- 4766 |
| SEQ41 | CPR37 | AGATTGTTGATGGACGTCTGG | Direct | 6982- 7002 |
| SEQ42 | CPR38 | TGACTCTCTGGCCCTTTCTT | Reverse | 8587- 8606 |
| SEQ43 | CPR39 | ATGCTCACAACAACGGAACA | Direct | 8616- 8635 |
| SEQ44 | CPR44 | GCCAAGTCACACACCAACAC | Direct | 8511- 8530 |
| SEQ45 | CPR47 | TCAAGTCGCTCAAAGAACGAT | Direct | 6959- 6979 |
| SEQ46 | CPR48 | CTTCCAACCCTGATGTCGAG | Direct | 5741- 5760 |
| SEQ47 | CPR49 | AGTTGGTCGTCGTGCTTGTA | Reverse | 6424- 6443 |
| SEQ48 | CPR50 | GCGATTCGCTCTTGAATGA | Reverse | 6554- 6572 |
| SEQ49 | CPR55 | ATGGGCGAAATATGTCGAAG | Direct | 6863- 6882 |
| SEQ50 | CPR56 | TCGTTGTGTTGTTGCTATTCG | Reverse | 7140- 7160 |
| SEQ51 | CPR57 | TCCAGTCAGTCGCCTTTTCT | Direct | 7335- 7354 |
| SEQ52 | CPR58 | GTGGCGAAAGACGAAAAGAC | Reverse | 8352- 8371 |
| SEQ53 | CPR60 | CCTTTCTCTCGCATCCTGAC | Reverse | 7891- 7910 |
| SEQ54 | CPR61 | TTCCTGTGTTTCCGTCACCT | Direct | 7182- 7201 |
| SEQ55 | CPR64 | CAAGTCGACTGGGAAAGGTC | Direct | 3076- 3095 |
| SEQ56 | CPR65 | CGCTGAAGAGAGGATTCGAG | Direct | 3476- 3495 |
| SEQ57 | CPR66 | GGAAAGAGAAAAGAAGCGAACA | Reverse | 5186- 5207 |
| SEQ58 | CPR67 | CGCTCTTGAATGAATCCTCTG | Reverse | 6546- 6566 |
| SEQ59 | CPR68 | ACTCGACCAACAACGAGAGC | Direct | 7682- 7701 |
| SEQ60 | CPR69 | TCGGTCCAGATACTTGCAGA | Direct | 8221- 8240 |
| SEQ61 | CPR70 f | ATGTTGGCCGAGTCAAAGAA | Direct | 6907- 6926 |
| SEQ62 | CPR71 r | GCATACTGTGCCTTGGGTCT | Reverse | 3726- 3745 |
| SEQ63 | CCPR01 | ACACGAGGGACGAGACTCC | Reverse | 4944- 4962 |
| SEQ64 | CCPR02 | ACGATGACAAGTCAATGGAAGA | Direct | 5398- 5417 |
| SEQ65 | CCPR03 | GTACGACGCCGTTTTCAGAC | Direct | 5877- 5896 |
| SEQ66 | CCPR04 | GCCGGTCTGGTAAGTGATTC | Reverse | 5702- 5721 |
| SEQ67 | CCPR05 | TGGGCACAAACTACCTGTTG | Direct | 6290- 6309 |
| SEQ68 | CCPR06 | TTGCCGTTCCCGTTAGAATA | Reverse | 4546- 4565 |
| SEQ69 | CCPR07 | GCTGAAGTCAAGTCGCTCAA | Direct | 6952- 6971 |
| SEQ70 | CPR1exA | GCCACACTCTCCGATCTTGT | Direct | 4396- 4415 |
| SEQ71 | CPR1exB | GTCAGCAGAACCCTCCTTCA | Direct | 4360- 4379 |
| SEQ72 | CPR1exC | GCCACACTCTCCGATCTTGT | Direct | 4396- 4415 |
| SEQ73 | CPREx1fwd | GTCAGCAGAACCCTCCTTCA | Direct | 4360- 4379 |
| SEQ74 | CPREx1fwdb | TCAGGACCCTGTACAGTCAGC | Direct | 4345- 4365 |
| SEQ75 | CPRfwdC | GCACAGGAAGTTGGTTGGAT | Direct | 3805- 3824 |
| SEQ76 | CPRfwdD | CCACTAGGCAAGCCTCCA | Direct | 424-441 |
| SEQ77 | CPRfwdE | TGGAGAGATCCACAAGAAAAGG | Direct | 656-677 |
| SEQ78 | CPRfwF | ATAATGGCGAGCATCAGACC | Direct | 970-989 |
| SEQ79 | CPRfwG | AATCGGAAAAACACTGACAGC | Direct | 1244- 1264 |
| SEQ80 | CPRfwH | AACGCTCCAAAGATGACGAC | Direct | 1701- 1720 |
| SEQ81 | CPRfwi | GGTCCTGGATGGTATCATGG | Direct | 2647- 2666 |
| SEQ82 | CPRfwJ | ATCTGAGGGGCGACAGAGTA | Direct | 1604- 1623 |
| SEQ83 | CPREx1rev | TCCCAACAGTCGATCCTTGT | Reverse | 4457- 4476 |
| SEQ84 | CprEX1REVB | ATCCAAGGGCGAGGAGAG | Reverse | 4433- 4450 |
| SEQ85 | CprEX1REVc | ACAGGGTCCTGACTGACCTG | Reverse | 4337- 4356 |
| SEQ86 | CprEX1REVd | CGAGAGAGACGAGAGAGGATG | Reverse | 4292- 4312 |
| SEQ87 | CPRrevE | CGATCAACAAACACGCAGAC | Reverse | 3964- 3983 |
| SEQ88 | CPRrevF | TCGCCCTTGGATTCTATAACA | Reverse | 4439- 4459 |
| SEQ89 | CPRrevg | CGTATGTATCCGCACAAGCA | Reverse | 3661- 3680 |
| SEQ90 | CPRrevH | CCTCAAACATCTCCTCTCATCC | Reverse | 3254- 3275 |
| SEQ91 | CPRrevi | TCAGGACTGCCTCTCCAGAT | Reverse | 2852- 2871 |
| SEQ92 | CPRrevJ | AGGTGCCGCTCTCTCTTTCT | Reverse | 2367- 2386 |
| SEQ93 | CPRrevK | CCCAATCGTCTTTGTCGTTT | Reverse | 2032- 2051 |
| SEQ94 | CPRrevL | TTTGACACCCAAAGTCTTTCC | Reverse | 2420- 2440 |
| SEQ95 | CPRrevM | TTTCGGCTTCTTCGAATGTC | Reverse | 3008- 3027 |
| SEQ96 | CPRatg | ATGGCCACACTCTCCGATCT | Direct | 4393- 4412 |
| SEQ97 | CPRstop | CTACGACCAGACGTCCATCA | Reverse | 6989- 7008 |
| SEQ98 | CPR wt fw | TTTCCTTCCTCTCCTCATCG | Direct | 5203- 5222 |
| SEQ99 | CPR WT REV2 | AGGGAACGTGTGTGACGTTT | Reverse | 8780- 8799 |
| SEQ100 | CPR WT REV | CAGGAAACAAGGGAACGTGT | Reverse | 8789- 8808 |
| SEQ101 | mCPR-F | CAGCAGAACCCTCCTTCAGT | Direct | 4362- 4381 |
| SEQ102 | mCPR-F2 | CCTTCAGTATCCAATTAAAATGGC | Direct | 4374- 4397 |
| SEQ103 | mCPR-R | GGAAAGCATGGAAAAGAGGA | Reverse | 7051- 7070 |
| SEQ104 | mCPR-R2 | GACGGAAACACAGGAAAGGA | Reverse | 7178- 7197 |
| SEQ105 | RTCPR-F | TGGATCCAAGCCCACATATT | Direct | 4530- 4549 |
| SEQ106 | RTCPR-R | GGAACCTCGGTTACGACAAA | Reverse | 5506- 5525 |

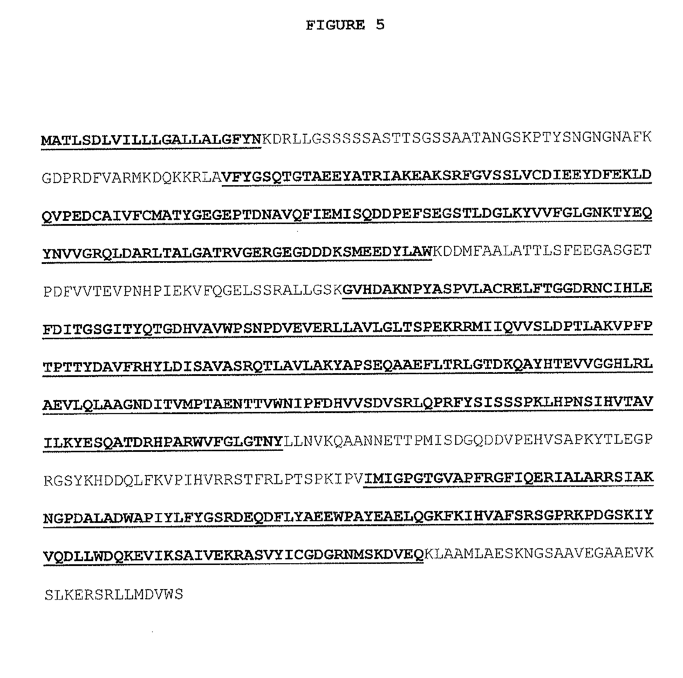

On the other hand, this invention relates to an amino acid sequence with cytochrome P450 reductase activity, corresponding in its preferred embodiment as described in the SEQ3 (Figure 5) and encoded by the CRTR gene, RNA messenger or complementary DNA generated from that gene, the complementary strands, allelic variants and sequences represented by SEQ1 or SEQ2 with addition, insertion, deletion and/or replacement of one or more nucleotides, or nucleic acid sequences hybridized under standard stringency conditions with the genomic sequence or complementary DNA (eg Detailed Conditions Sambrook et al. 2001). In its preferred embodiment, the amino acid sequence of CPR activity polypeptide has 746 amino acids, SEQ3 (Fig. 5), by comparisons using bioinformatics tools, we identified certain conserved domains in the SEQ3 marked, and correspond, in order of occurrence to: transmembrane domain, FMN binding domain, FAD binding domain and binding domain of NAD (P) H.

The present invention is also directed to SEQ3 polypeptide variants. These variations relate to the addition, insertion, deletion and/or replacement of one or more amino acids in those sequences, as these derivatives retain the activity described. This activity can be measured by any test known in the art or specifically described herein. Such variants can be synthesized by chemical synthesis or by recombination based on DNA sequences available, using state of the art methods.

The present invention relates to the use of CPR polypeptide, which is the SEQ3 preferred embodiment, for obtaining modified organisms for purposes of increasing the production of astaxanthin or generate new metabolic pathways leading to its bio manufacturing in appropriate agencies. In another embodiment, using the DNA sequence coding for the sequence SEQ3 amended to delete the role cytochrome P450 reductase in astaxanthin-producing organisms in conjunction with astaxanthin synthase, the suggested use is for research purposes.

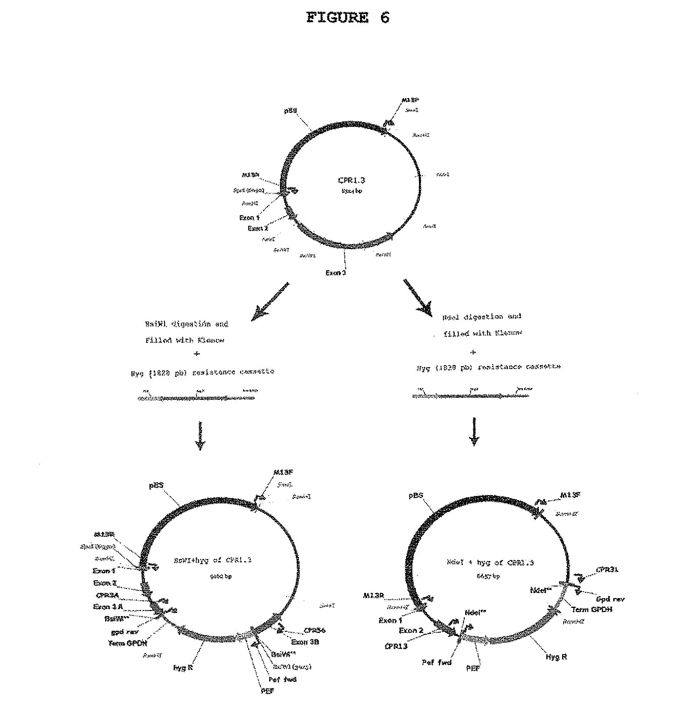

In its preferred embodiment, the invention includes the deletion of the crtR X gene function in yeast. dendrorhous and evaluation of the strains transformed phenotypic and genotypic. crtR gene deletion is performed by replacing the native gene in wild strains of X. dendrorhous by a module that has a fragment of this gene interfered by means of a selection marker, preferably antibiotic resistance genes. In its preferred embodiment the native gene replacement by homologous recombination was performed using a suitable vector containing the module described above, in particular, were designed vectors described in Figure 6 and turned them two strains of X. native dendrorhous obtaining two transformed strains (CBSTr and T13) that have a reduced capacity for production of astaxanthin from the native strains from. The phenotypic evaluation of the transformed strains was done by visual inspection of the color of yeast in culture and its comparison with the native strain in another way, by identifying the pigments it contains, preferably using the HPLC (High Performance Liquid Chromatography) technique.

In genome-wide assessment of gene deletion strains transformed crtR in this invention includes the analysis and comparison of the genomic sequence for this gene using molecular biology techniques present in the state of the art, preferably through their PCR amplification using the fragments described in Table 1, including fragments SEQ14, SEQ15, SEQ20, SEQ30, SEQ35, SEQ50, SEQ76, and those described in Table 3.

The present invention is also directed to a vector or plasmid comprising the DNA sequences described above and a host cell transformed or transfected with said DNA or vector or plasmid described above.

Furthermore, this invention provides compositions, products and/or formulations comprising such vectors or plasmids, genomic DNA, cDNA and gene fragments previously described. Such compositions, products and/or formulations, as well as the sequences described above, can be used for research, production, analysis or molecular biology techniques existing in the state of the art. Preferably in the processes required to transform organizations in order to obtain a modified production of astaxanthin and evaluation of this transformation.

The present invention contemplates the production of recombinant organisms transformed from vectors or plasmids and DNA they contain and the culture of these organisms processed under conditions that lead to the production of said polypeptide or production of astaxanthin.

The polypeptide referred to herein and encoding sequences that are useful to genetically manipulate organisms naturally producing astaxanthin to enhance their capacity to generate biosynthetic or biosynthetic pathway in other organisms, whose growth qualities, culture conditions, bioavailability and other may be more attractive products to obtain the pigment, as is the case of *Saccharomyces cerevisiae.*

Here are some application examples, not intended to be exclusive or limiting to the invention.

### APPLICATION EXAMPLES

### Example 1: Obtaining the genomic DNA sequence of the gene from X. ACWP dendrorhous.

To isolate and characterize the gene X ACWP dendrorhous oligonucleotides were designed to amplify parts of the gene allowing a CRTR by PCR reaction (Polymerase Chain). To this end we compared the nucleotide sequences of genes of enzymes CPRs of yeast *Cunninghamella echinulata, Saccharomyces,* and *Rhodotorula minuta* by Clustal W program were identified and conserved regions were designed based on these semi-degenerate oligonucleotides described in Table 1 (SEQ4 to SEQ9) and whose location within the compared sequences are shown in Figure 3. Using a pair of primers and SEQ8 SEQ4, CRTR gene was amplified from genomic DNA of X. dendrorhous wild strain UCD 67-385, using a PCR protocol under the following conditions: initial denaturation 95°C for 3 minutes, 35 cycles: denaturation at 94° C for 30 seconds, annealing at 55°C for 30 seconds extension at 72°C for 3 minutes. After a final extension at 72°C for 10 minutes. The obtained amplified were run in agarose gel 0,8% and purified from it, then sequenced. Using this technique it was possible to obtain an amplicon of 1649 base pairs. By comparing the sequence obtained with international data bases using the BLASTX program (Search protein database using the nucleotide sequence obtained translated into amino acids) was found to be amplified and had obtained a high percentage of similarity with other yeast CPRs, being the biggest the *Trametes versicolor* BAB83588.1 identities (identity: 61% and value of E: 3x10⁻¹⁴²) and *Phanerochaete chrysosporium* AAG31351.1 (identity: 59% and E value: 1x10⁻¹³⁷).

From the sequence obtained was analyzed those conserved coding segments (using BlastP program) and based on them designed new specific primers for the CRTR gene X. dendrorhous (SEQ10 to SEQ13, Table 1). These primers were used to find clones in a genomic library of X. dendrorhous built into the BamHI site of plasmid used as a guest YIp5 the DH5 strain of E. coli, containing genomic DNA segments coding for the CRTR gene. The pursuit of these clones was performed by amplification by PCR of the inserts of the genomic library clones. CRTR gene contains BamHI sites in their sequence, therefore it was not possible to obtain a clone containing the full CRTR gene from the gene library used. However, we obtained two clones containing parts of the gene. One with an insert 12820 bp was completely sequenced and contains the gene ACWP from the base nucleotide No. 82 of exon 1 to the end of the gene, the other clone contains an insert of approximately 6500 bp was completely sequenced and the promoter region of the gene ACWP and 88 bp exon 1. By digestion with restriction enzymes and hybridization tests, using genomic DNA as a probe a fragment amplified with primers SEQ8 SEQ4 and determined that the CRTR gene does not contain SalI sites in sequence and a SalI fragment of approximately 4900 bp containing the gene in its entirety. Basically, the protocol used for these experiments was the following: The genomic DNA of strain X-385 UCD67 dendrorhous, was digested with different restriction enzymes and electrophoresed on agarose gel 0.8%. Then the DNA was denatured and transferred to a nylon membrane and hybridization was performed using a DNA fragment corresponding to part of the CRTR gene as a probe. Preparation of membranes and hybridization was performed according to standard methods to Sambroock et al., 2001.

Based on this background, we built a new partial genomic library using SalI DNA fragments of X. dendrorhous selected sizes between approximately 6400 and 4400 bp, was used as vector pBluescript SK + and E. coli strain DH5 as a host, the development of this new genomic library was performed according to the manufacturer's instructions. Using this new gene library, inserts were amplified by PCR using primer pairs and SEQ13 SEQ10. In this way we obtained a clone containing the gene entirely CRTR (SEQ1).

### Example 2: Preparation of complementary DNA sequence of the gene from X. ACWP dendrorhous.

To Obtain the DNA sequence complementary to the CRTR gene, we used a library of complementary DNA of X. dendrorhous, from which were sequences amplified by PCR using primers pairs and SEQ13 SEQ10 that hybridizes with the gene coding segments whose amplicon size expected from the CRTR gene cDNA is 338 bp. The cDNA library was constructed using the commercial kit "pBluescript II XR cDNA library construction kit from Stratagene, using as host the XL10-Gold strain of *Escherichia coli*, according to the manufacturer's instructions. The conditions used for PCR reactions were: initial denaturation at 95°C during 3 minutes, 35 cycles: denaturation at 94°C during 30 seconds, annealing at 55°C during 30 seconds, extension at 72°C for 3 minutes. After a final extension at 72°C for 10 minutes. The amplicons obtained were loaded onto an agarose gel 0,8% positive clones identified mixtures for amplification of cDNA CRTR gene.

This technique identified a clone with an insert of CRTR gene cDNA. This insert was sequenced, obtaining a sequence of 2.241 base pairs (SEQ2).

Comparing the cDNA sequence obtained from the genomic DNA sequence of the gene it determined the organization of exons and introns (Figure 2).

Later the translation into amino acid sequence obtained (SEQ2), using the software package Vector NTI Suite 10 (Informax), after having determined the correct reading frame were identified maintained domains of the protein described in Figure 5 , in the appearance sequence, they are transmembrane domain, FMN binding domain, FAD binding domain and binding domain NAD (P) H. The identification of the transmembrane domain was performed using the program TMpred (www.ch.embnet.org/software/ TMPRED-form.html), obtaining a score of 2.280, consecutive domains were identified by InterProScan program (http://www.ebi .ac.uk/InterProScan/), with values of E 2,5 x10⁻⁴¹, 1,6 and 7,8 x10⁻⁶⁷ x10⁻²³ respectively.

Example 3. The determination of the functional importance of CPR polypeptide encoded by the gene CRTR, along the X carotenogenic dendrorhous.

To demonstrate the importance of the CRTR gene in the biosynthetic pathway of astaxanthin in yeast X dendrorhous were constructed gene deletion mutants in strains of X. ACWP dendrorhous wild and compared the phenotypes and genotypes of the mutants with the original strain. To this end, it was inserted into a transformation vector, a construction which it replaced a DNA fragment containing the gene for a module CRTR resistance to the hygromycin B antibiotic. This module consists of antibiotic resistance gene to hygromycin B (hph) under the control of the TEF gene promoter (which encodes the translation elongation factor of, EF-1α) and GPD gene terminator region of X. dendrorhous.

Figure 6, shows the detail of the constructs made, basically the CPR1.3 vector has a DNA fragment of X. dendrorhous, content segment between nucleotides 4531 and 9902 of the SEQ1, was digested with restriction enzymes BsiWI and NdeI to generate a deletion in the gene CRTR. The ends of the fragment obtained (containing the deletion CRTR), were filled with Klenow polymerase to enable the module linked hygromycin resistance whose ends are blunt. Thus obtained two clones: CPR1.3ΔNdeI CPR1.3ΔBsiMI + and + hyg hyg. The insertion of this construct into the genome of wild strains used, was performed by homologous recombination technique detailed below. Using these vectors wild strains of X. dendrorhous were transformed: UCD 67-385 and CBS 6938.

UCD67-385 strain was transformed with a linear DNA fragment containing the CRTR gene deletion and resistance to hygromycin module. This fragment was obtained from the digestion of clone CPR1.3ΔBsiWI + hyg with the endonucleases SmaI and SpeI. By a double homologous recombination event, an allele of strain UCD67 ACWP-385 was replaced by deletion, resulting in strain T13.

The strain CBS6938 was transformed with the clone CPR1.3ΔNdeI + hyg (circular DNA) containing the CRTR gene deletion and resistance to hygromycin module. By an homologous recombination event, was replaced with wild CRTR gene mutated gene, yielding strain CBSTr

The transformation of both wild-type strains was performed by electrophoration, according to the protocols described by Adrio et al. (1995) and Kim et al. (1998). Briefly, inoculated 200 ml YM medium business (glucose 1%, 0.3% yeast extract, malt extract 0,3% and 0,5% bacto-peptone) with 1-2 ml of a culture of X. dendrorhous 48 hours of growth and is incubated with constant stirring at 22°C until a DO₅₅₀ₙₘ of 1,2. The cells were harvested and resuspended in 25 ml of a BD buffer (50 mM potassium phosphate buffer, pH 7,0, 25 mM ditiotritol [DTT]) and incubated at 22°C for 15 minutes. The cells were washed twice with 25 ml of STM buffer (270 mM sucrose, 10 mM Tris-HCl, pH 7,5, 1 mM MgCl 2) cold and resuspended in 1 ml of STM buffer. To transform mixing 60 µl of electrocompetent cells with 10 to 20 micrograms of transforming DNA in a volume of 5 µl. The electrophoration conditions are: 125 mF, 600 Ω, 0.45 kV and then the cells were resuspended in 1 ml of liquid YM and incubated at 22°C for 5 hours. Finally, cells were grown in YM medium plates and agar 2% supplement with 10 ug / ml of hygromycin B. Using this method of selection clones were identified which have the insert with the hygromycin resistant gene.

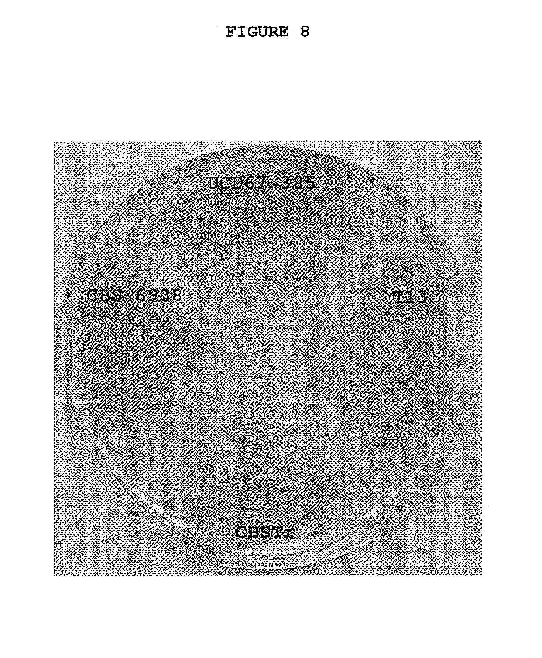

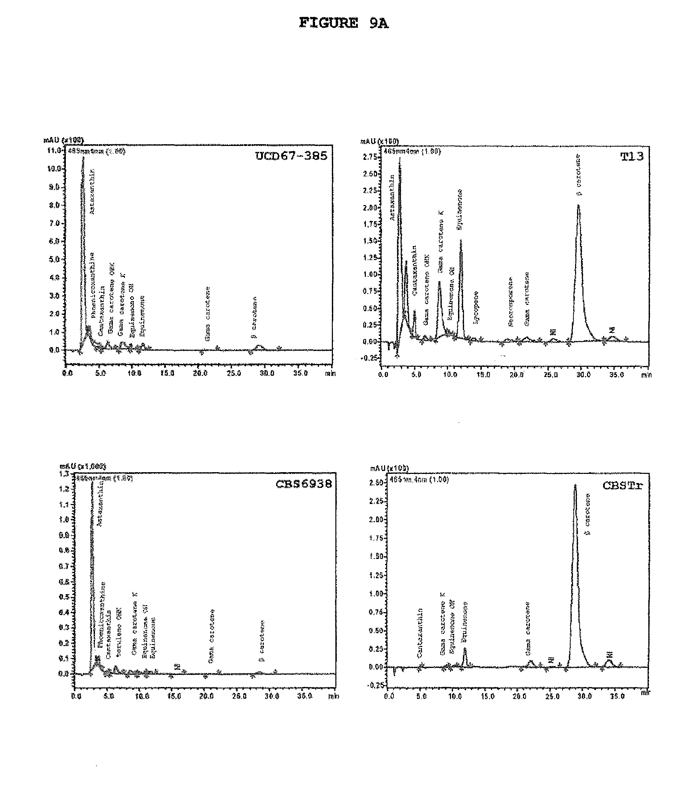

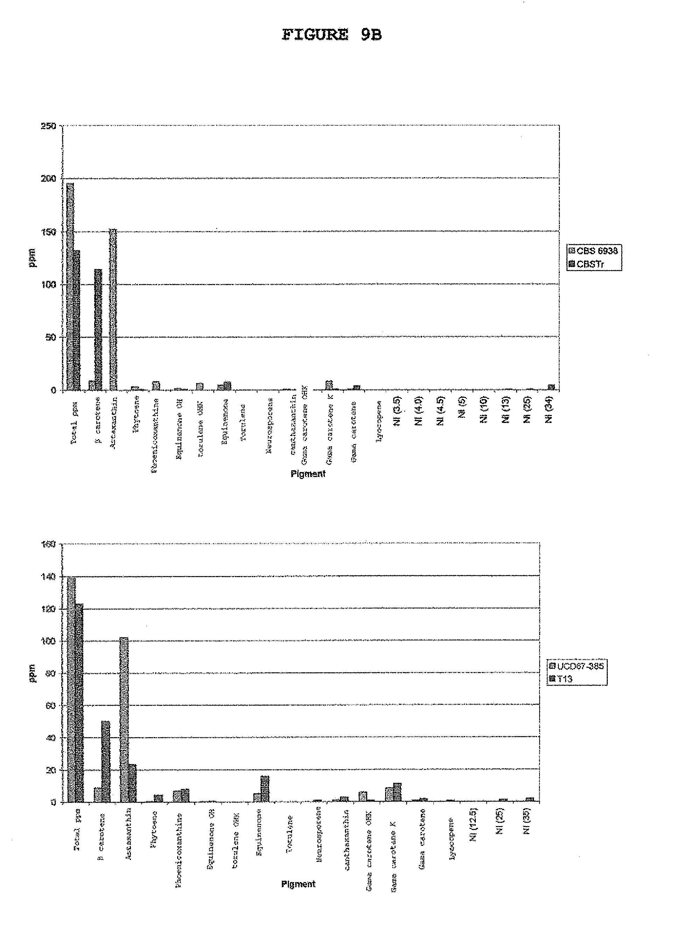

Of the obtained clones in the first selection with antibiotics, two clones were chosen transformants of each of the wild strains. After the transformation of the strain CBS 6938 a yellow strain (CBSTr) was obtained, which color indicates that it accumulates beta-carotene and is unable to synthesize astaxanthin. By transforming the strain UCD 67-385, a transformant pale (T13) was obtained, which also indicates the pathway of synthesis of astaxanthin is affected (phenotypic evidence shown in Figure 8). Total pigments were extracted from 2 wild strains and 2 strains transformants and its composition was analyzed by HPLC (Fig. 9), the following protocol based on the method of An et al. (1989) : A cell pellet from 5 to 20 ml of culture was added between 1 and 3 ml of acetone plus one volume of glass beads, which was then agitated at full vortex speed for 1 min. The organic phase was recovered by centrifugation 12,100 xg at 4°C. total pigments were extracted with petroleum ether, then dried with nitrogen gas, dissolved in acetone and separated by HPLC with a reverse phase column, LiChroCART RP-18 125-4 (Merck) using acetonitrile: methanol: isopropanol (85:10:5 v/v) as mobile phase with a flow of 1 ml/min at room temperature in isocratic conditions. The spectrum of each peak of circumvention were recovered by a diode array detector. The caretonoids were identified according to its absorption spectrum, retention time and comparison with specific standards. It was noted that the composition of carotenoids in transformants of strains is quite different from the wild strains from which they come. The transformant CBSTr accumulates beta-carotene and is unable to synthesize astaxanthin, unlike the wild strain from which it comes (CBS 6938), which produces astaxanthin and a minimum amount of beta-carotene. The T13 transforming their production of astaxanthin, reduced by 75% approximately the cost of increased production of beta-carotene, Unlike the wild strain from which it comes (UCD67-385), which produces high amounts of astaxanthin and minimal amounts of Beta-carotene (Figure 9).

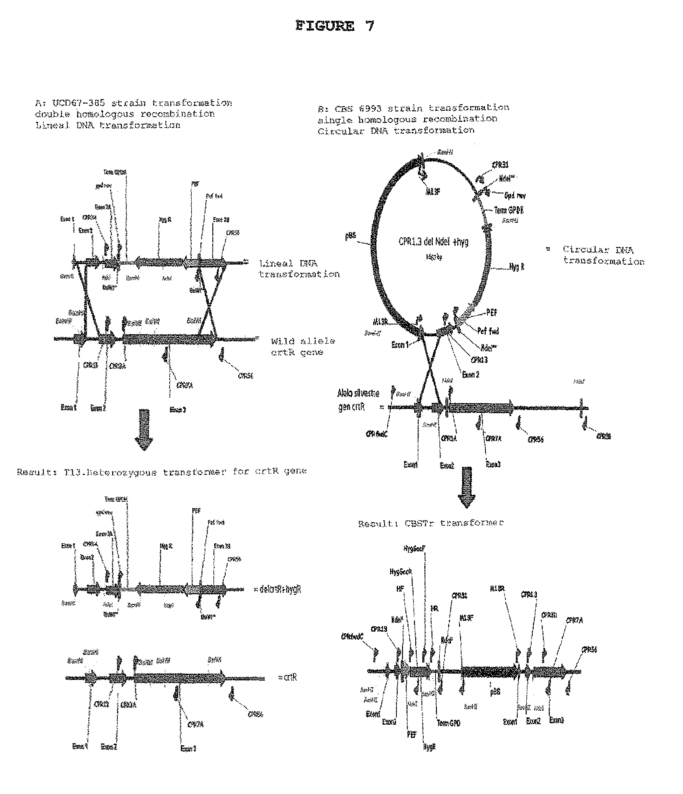

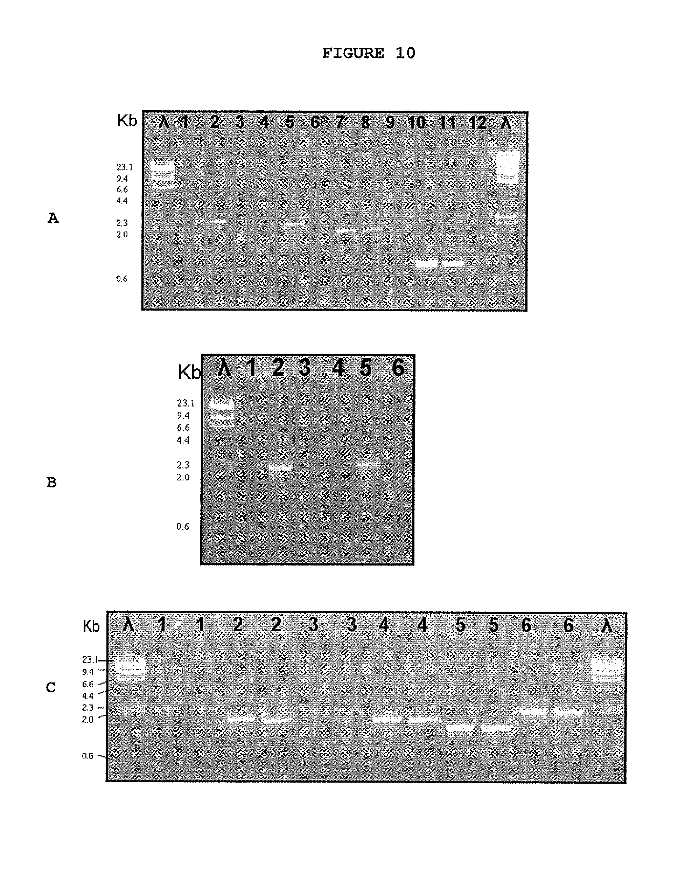

To confirm the genome-wide transformation of the strains mentioned above, we examined the locus affected by their inserts amplified by PCR, using as template, genomic DNA from the transformants and the wild strains. For the analysis of the transformed strain T13, PCR reactions were performed with the following pairs of primers: gdp + SEQ50 rev, fwd SEQ14 + pef, SEQ14 + SEQ50 and used as the wild strain hardened UCD 67-385, the transformed strain T13 and distilled water as negative control. For the analysis of the transformed strain CBSTr PCR reactions were performed with the following pairs of primers: fwd + SEQ35 pef, rev + SEQ30 gpd and using a mild strain CBS 6938 wild, the transformed strain CBSTr and distilled water as negative control also used primers pairs: hygSecR SEQ75 +, HF + SEQ35, HygSecF + M13F, M13R + SEQ15, SEQ20 + + SEQ50 SEQ30 SEQ50 and using as template, the transformed string CBSTr. Some of these primers do not hybridize with the sequence of ACWP, their sequence is detailed in Table 2 and their relative location is shown in Figure 7. PCR reactions were performed according to the following protocol: initial denaturation 95°C for 3 minutes, 35 cycles: denaturation at 94°C for 30 seconds, annealing at 55°C for 30 seconds, extension at 72°C for 3 minutes. After a final extension at 72°C for 10 minutes and were loaded on agarose gels 0,8%. The results obtained are shown in Figure 10 and comparison between the sizes of expected amplified and those obtained are shown in Table 3.

**Table 2: No sequences complementary splitters to ACWP, used in genomic analysis of transformed strains**

| **Primer** | **Sequence 5' - 3'** | **Orientation (Hybrid)** |
|---|---|---|
| Pef fwd | GATATCGGCTCATCAGCCGAC | Direct (Promoter Pef) |
| gpd rev | ATGAGAGATGACGGAGATG | Reverse (terminator GPDH) |
| HR | CTATTCCTTTGCCCTCGGAC | Reverse(gen resistanse to higromicine) |
| HF | ATGAAAAAGCCTGAACTCACC | Direct (gen resistance to higromicina) |
| HygSecR | GTATTGACCGATTCCTTGCG | Reverse (gen resistencia higromicina) |
| HygSecF | TCGCCAACATCTTCTTCTGG | Direct (gen resistencia higromicina) |
| M13R | GGAAACAGCTATGACCATG | Reverse (plasmido pBS) |
| M13 F | TGTAAAACGACGGCCAGT | Direct (plasmido pBS) |

**Table 3: Size of amplified expected and obtained in the analysis of transformants T13 and CBSTr**

| A. Results for the transformant T13 | | | | |
|---|---|---|---|---|
| **Pair of primers** | **Expected size *crtR*** | **Obtained size *crtR*** | **Expected size** | **Obtained size** |
| | **wt (pb)** | **wrt (pb)** | **Δ*crtR*+hyg R** | **Δ*crtR*+hyg R** |
| gpd rev + SEQ50 | / | / | 2.380 | 2.400 |
| SEQ14 +Pef fwd | / | / | 2.208 | 2.200 |
| SEQ14 + SEQ50 | 1.844 | 1.800 | 2.682 | / |
| SEQ14 + SEQ15 | 808 | 800 | / | / |

| B. Results for transforming CBSTr | | | | |
|---|---|---|---|---|
| Primer pairs | | Amplified expected size (pb) | | Amplified obtained size (pb) |
| Pef fwd - SEQ35 | | 2.060 | | 2.000 |
| Gpd rev + SEQ30 | | 2.154 | | 2.200 |
| SEQ75 + hygSecR | | 2.269 | | 2.200 |
| HF + SEQ35 | | 1.667 | | 1.700 |
| HygsecF + M13F | | 2.113 | | 2.100 |
| M13R + SEQ15 | | 1.696 | | 1.700 |
| SEQ20 + SEQ50 | | 1.376 | | 1.400 |
| SEQ30 + SEQ50 | | 2.155 | | 2.100 |

The sizes of amplicons obtained from the transformed strains match the expected results (Table 3). This analysis was confirmed by DNA-DNA hybridization. Hybridization experiments were performed by digesting genomic DNA from wild strains (UCD67-385 and CBS6938) and transformant strains (T13 and CBSTr) with different restriction endonucleases. Digests were run on agarose gels 0,8% and then denatured and transferred to nylon membranes for hybridization. The membranes were hybridized separately with two probes: 1)a gene probe amplified with primers SEQ4 CRTR gene and SEQ8, 2) hygromycin gene probe. Preparation of membranes and hybridization was performed according to standard methods Sambroock et al., 2001.

The results indicate the T13 transformant is heterozygous for the CRTR gene, where a wild and a mutant allele for a gene dosage effect, this transformant produces less astaxanthin and beta-carotene accumulates more than its parental wild with these results shows that the insertion of the gene deletion CRTR with hygromycin B resistance corresponds to the schematic in Figure 7. In the case of transforming CBSTr, a copy of CRTR gene was mutated and therefore is unable to produce astaxanthin, beta-carotene accumulating. This result indicates that the strain CBS6938 is haploid, at least for the CRTR gene. All amplicons were fully sequenced and their analysis confirmed their origin

### Reference:

Adrio J., Veiga M. 1995. Transformation of the astaxanthin-producing yeast Phaffia rhodozyma. Biotech. Techniques 9: 509-512.
Andrewes A., Starr M. 1976. (3R,3R')- Astaxanthin from the yeast Phaffia rhodozyma. Phytochem 15: 1009-1011.
Alcaino, J. 2002. Structural organization of the phytoene synthase gene in the genome of Xanthophyllomyces dendrorhous (formerly Phaffia rhodozyma). Thesis for the degree in Molecular Biotechnology Engineering. Universidad de Chile.
Alvarez V, Rodriguez-Saiz M, de la Fuente JL, Gudina EJ, Godio RP, Martin JF, Barredo JL. 2006. The crts gene of Xanthophyllomyces dendrorhous encodes a novel cytochrome-P450 hydroxylase involved in the conversion of beta-carotene into astaxanthin and other xanthophylls. Fungal Genet Biol. 43:261-72.
An, G-H., D. B. Schuman, y E. Johnson. 1989. Isolation of Phaffia rhodozyma mutants with increased astaxanthin content. Appl. Environ. Microbiol. 55: 116-124.
Cunningham, F. X. y E. Gantt. 1998. Genes and enzymes of carotenoid biosynthesis in plants. Annu. Rev. Plant Physiol. Plant Mol. Biol. 49: 557-583.
Higuera-Ciapara, I, Félix-Valenzuela L., Goycoolea FM: Astaxanthin: a review of its chemistry and applications. Crit. Rev. Food. Sci. Nutr. 2006, 46:185-196.
Johnson, E.A., Conklin, D., Lewis, M.J., 1977. The yeast Phaffia rhodozyma as a dietary pigment source for salmonids and crustaceans. J. Fish. Beast. Board Can. 34, 2417-2421.
Kajiwara, P., P. D. Fraser, K. Kondo, y N. Misawa. 1997. Expression of an exogenous isopentenyl diphosphate isomerase gene enhances isoprenoid biosynthesis in Escherichia coli. Biochem. J. 324: 421-426.
Kim I., Nam S., Sohn J., Rhee S., An G., Lee S. y Choi E. 1998. Cloning of the ribosomal protein L41 gene of Phaffia rhodozyma and its use as a drug resistance marker for transformation. Appl. Environ. Microbiol. 64:1947-1949.
Linden H. 1999. Carotenoid hydroxylase from Haematococcus pluvialis: cDNA sequence, regulation and functional complementation. Biochim Biophys Acta 1446: 203-212.
León, R. 2000. Characterization of genetic determinants of the synthesis of astaxanthin in Xanthophyllomyces dendrorhous (formerly Phaffia rhodozyma). Thesis for the degree of Doctor of Science c/m in Biology. Universidad de Chile.
Margalith PZ. 1999. Production of ketocarotenoids by Microalgae. Appl Microbiol Biotechnol. 51 (4) :431-8.
Niklitschek, M., J. Alcaíno, S. Barahona, D. Sepúlveda, M. Carmona, A. Wozniak, A. Marcoleta, P. Lodato, M. Baeza, V. Cifuentes. 2008. Genomic organization of the structural gene controlling the astaxanthin biosynthesis pathway of Xanthophyllomyces dendrorhous. Biol Res, In press. (MS 05-512).
Ojima K, Breitenbach J, Visser H, Setoguchi Y, Tabata K, Hoshino T, van den Berg J, Sandmann G. 2006. Cloning of the astaxanthin synthase gene from Xanthophyllomyces dendrorhous (Phaffia rhodozyma) and Its assignment as a beta-carotene 3-hydroxylase/4-ketolase. Mol Genet Genomics. 275 (2) :148-58.
Sambroock, J., Russell, D, W. 2001. Molecular cloning. A laboratory manual, 3rd ed. Cold Spring Harbor Laboratory Press, Cold Spring Harbor, NY.
Sommer, TR, Potts WT, Morrissy, NM, 1991. "Utilization of microalgal astaxanthin by rainbow trout Oncorhynchus mykiss. Aquaculture 94, 79-88.
van den Brink HM, van Gorcom RF, CA van den Hondel and PJ Punt. 1998. Cytochrome P450 enzyme systems in fungi. Fungal Genet Biol 23: 1-17.
Green, JC, P. Krubasik, G. Sandmann, and AJJ van Ooyen. 1999a. Funtional Isolation and Characterisation of a novel type of carotenoid biosynthetic gene from Xanthophyllomyces dendrorhous. Mol. Gen. Genet. 262: 453-461.
Green, JC, N. Misawa, and AJJ van Ooyen. 1999b. Cloning and characterization of the astaxanthin biosynthetic gene encoding phytoene desaturase of Xanthophyllomyces dendrorhous. Biotechnol. Bioeng. 63: 750-755.

## Claims

1. A DNA sequence **CHARACTERIZED in that** the DNA sequence encodes an enzyme having cytochrome P450 reductase activity that acts as electron donor for the reaction catalyzed by the enzyme that converts astaxanthin synthase and beta-carotene into astaxanthin, preferably into *X. dendrorhous.*

2. The isolated DNA according to claim 1, **CHARACTERIZED in that**:
a) the nucleotide sequence codes for said enzyme and has a sequence as shown in SEQ1, or
b) A nucleotide sequence, because its genetic code is degenerated, and encodes cytochrome P450 reductase having the same amino acid sequence encoded by the nucleotide sequence of a);
c) A nucleotide sequence hybridising the complement of the nucleotide sequence of a) and b) under standard stringency conditions.

3. The isolated DNA according to claim 1, **CHARACTERIZED in that**:
a) A nucleotide sequence is represented by SEQ2;
b) A nucleotide sequence, which because the genetic code is degenerated, encodes cytochrome P450 reductase having the same amino acid sequence encoded by the nucleotide sequence a);
c) A nucleotide sequence hybridizing the complement of the nucleotide sequence of a) and b) under standard stringency conditions.

4. A sequence of amino acids that have cytochrome P450 reductase activity, encoded by the nucleotide sequences of claims 2 and 3, **CHARACTERIZED in that** the sequence SEQ3, is preferably *X. dendrorhous.*

5. The isolated polypeptide according to claim 4 **CHARACTERIZED in that**:
a) An amino acid sequence represented by SEQ3, or
b) A polypeptide similar to SEQ3 that changes into one or more amino acids and which maintains the cytochrome P450 reductase activity described.
c) A polypeptide as described in a) and b) synthesized by chemical synthesis or bio-synthesis.

6. An oligonucleotide **CHARACTERIZED in that** it hybridizes with the sequences corresponding to claims 1 to 3.

7. An isolated oligonucleotide according to claim 6, **CHARACTERIZED in that** it is useful for amplifying DNA sequences by PCR or other use of molecular biology techniques described in the state of the art.

8. A vector or plasmid **CHARACTERIZED in that** it comprises the DNA segment claimed in claims 1 to 3.

9. A host organism **CHARACTERIZED in that** it is transformed or transfected by the isolated DNA described in claims 1 to 3 or a vector or plasmid described in claim 7.

10. A process for producing a polypeptide having cytochrome P450 reductase activity **CHARACTERIZED in that** it comprises culturing a host organism transformed as described in claim 8, grown under conditions conducive to the production of said enzyme.

11. A process for the biological production of astaxanthin **CHARACTERIZED in that** it comprises the introduction of one or more isolated DNA as detailed in the claims from 1 to 4 in a suitable host organism, grown under conditions conducive to the production of astaxanthin and the recovery of astaxanthin from the culture.

12. A process for the production of astaxanthin starting from Beta-carotene **CHARACTERIZED in that** it provides for the donation of electrons by the polypeptide described in claims 4 and 5 for the reaction catalyzed by astaxanthin synthase, in an appropriate reaction mixture containing an appropriate reconstituted membrane.

13. The process for the production of astaxanthin from beta-carotene **CHARACTERIZED in that** the polypeptide described in claims 4 and 5 is present in the form of a reconstituted membrane which is prepared from a biological membrane as a microsome or a mitochondrial membrane.

14. The process for the production of astaxanthin from beta-carotene CHARACTERIZED because the polypeptide described in claims 4 and 5 is presented in the form of a reconstituted artificial membranes such as a liposome.

15. The compositions, products and/or formulations **CHARACTERIZED in that** they comprise the vectors or plasmids, genomic DNA, DNA and CRTR gene fragments listed in claims 1, 2, 3, 6, 7, 8, useful for transforming host organisms and for the production of astaxanthin and covered in claims 9, 10 and 11.
